(19) Europäisches Patentamt
European Patent Office
Office européen des brevets

(11) **EP 2 168 591 A1**

(12) **EUROPEAN PATENT APPLICATION**

(43) Date of publication:
**31.03.2010 Bulletin 2010/13**

(51) Int Cl.:
*A61K 38/04* $^{(2006.01)}$  *C07K 7/08* $^{(2006.01)}$

(21) Application number: **08016808.1**

(22) Date of filing: **24.09.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(71) Applicant: **Helmholtz Zentrum München Deutsches Forschungszentrum für Gesundheit und Umwelt (GmbH) 85764 Neuherberg (DE)**

(72) Inventors:
• **Durner, Jörg**
  **85764 Oberschleißheim (DE)**
• **Lindermayr, Christian**
  **86316 Friedberg (DE)**

(74) Representative: **Vossius & Partner Siebertstraße 4 81675 München (DE)**

(54) **Antimicrobial peptides**

(57)    The present invention relates to the present invention relates to a peptide comprising or consisting of a sequence of formula I A-B-C-D-E-F (formula I), wherein A is a peptide consisting of three hydrophobic amino acid residues; B is an amino acid residue selected from the group consisting of hydrophobic and basic amino acid residues; C is a peptide consisting of two hydrophobic amino acid residues; D is a peptide consisting of two basic amino acid residues; E is a peptide consisting of two hydrophobic amino acid residues; and F is a peptide consisting of two basic amino acid residues; or a peptidomimetic thereof; wherein the basic amino acid residues are selected from the group consisting of arginine, lysine and histidine; wherein the hydrophobic amino acid residues are selected from the group consisting of leucine, alanine, valine, isoleucine, methionine and phenylalanine; and wherein said peptide or peptidomimetic has antimicrobial activity. Furthermore, the invention relates to a nucleic acid molecule encoding the peptide of the invention, a vector comprising the nucleic acid molecule as well as a host cell comprising the nucleic acid molecule or the vector. The present invention also relates to a method for producing the peptide of the invention, a composition as well as to the peptide of the invention for use in treating infectious diseases.

EP 2 168 591 A1

SEQUENCE LISTING

<110>  Helmholtz Zentrum München

<120>  Antimicrobial peptides

<130>  P2993 EP

<160>  4

<170>  PatentIn version 3.3

<210>  1
<211>  12
<212>  PRT
<213>  Artificial sequence

<220>
<221>  source
<223>  /note="Description of artificial sequence: antimicrobial peptide"

<400>  1

Leu Leu Ile Ala Ala Phe Lys Lys Leu Val Lys Lys
1               5                   10


<210>  2
<211>  12
<212>  PRT
<213>  Artificial sequence

<220>
<221>  source
<223>  /note="Description of artificial sequence: antimicrobial peptide"

<400>  2

Ala Leu Ala His Phe Leu Lys Lys Ala Ile Lys Lys
1               5                   10


<210>  3
<211>  12
<212>  PRT
<213>  Artificial sequence

<220>
<221>  source
<223>  /note="Description of artificial sequence: antimicrobial peptide"

<400>  3

Leu Leu Ile Lys Phe Leu Lys Arg Phe Ile Lys His
1               5                   10


<210>  4
<211>  12
<212>  PRT
<213>  Artificial sequence

<220>
<221>  source

```
<223>   /note="Description of artificial sequence: antimicrobial peptide"

<400>   4

Leu Leu Ile Arg Ala Ala Lys Lys Phe Ile Lys Lys
1               5                   10
```

**Description**

The present invention relates to the present invention relates to a peptide comprising or consisting of a sequence of formula I A-B-C-D-E-F (formula I), wherein A is a peptide consisting of three hydrophobic amino acid residues; B is an amino acid residue selected from the group consisting of hydrophobic and basic amino acid residues; C is a peptide consisting of two hydrophobic amino acid residues; D is a peptide consisting of two basic amino acid residues; E is a peptide consisting of two hydrophobic amino acid residues; and F is a peptide consisting of two basic amino acid residues; or a peptidomimetic thereof; wherein the basic amino acid residues are selected from the group consisting of arginine, lysine and histidine; wherein the hydrophobic amino acid residues are selected from the group consisting of leucine, alanine, valine, isoleucine, methionine and phenylalanine; and wherein said peptide or peptidomimetic has antimicrobial activity. Furthermore, the invention relates to a nucleic acid molecule encoding the peptide of the invention, a vector comprising the nucleic acid molecule as well as a host cell comprising the nucleic acid molecule or the vector. The present invention also relates to a method for producing the peptide of the invention, a composition as well as to the peptide of the invention for use in treating infectious diseases.

In this specification, a number of documents including patent applications and manufacturer's manuals is cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Antibiotics are among the most frequently prescribed medications in modern medicine and are agents with an activity against microorganisms such as bacteria, fungi or protozoa. The first antibiotic identified was penicillin, discovered accidentally from a mold culture. After the discovery of penicillin, many other naturally occurring antibiotics have been found so that today hundreds of different antibiotics are available to physicians for curing many illnesses ranging from minor discomforts to life-threatening infections.

Antibiotics usually act by either inhibiting the growth of a target microorganism or by killing it. Based on their target sites antibiotics may be classified as those acting, inter alia, on cell wall synthesis, membrane synthesis, transcription, translation or the respiratory chain. Furthermore, anti-bacterial antibiotics can be categorized based on their target specificity. Thus, "narrow-spectrum" antibiotics target particular types of bacteria, such as Gram-negative or Gram-positive bacteria, while broad-spectrum antibiotics affect a wide range of bacteria.

Recently, an increasing number of the antibiotics used for the treatment of infectious diseases have lost their effectiveness due to resistances developed by the microorganisms. Such resistances can be created by the target site itself such as, e.g., by modification of the site where the antibiotic interacts with the target components on/in the microorganism, or by acquiring resistance factors such as enzymes which degrade or modify the antibiotics thus depriving them of their antibiotic effect. Antibiotic resistance may also be due to reduced accumulation of the antibiotic in the microorganism caused by decreased drug permeability and/or increased active efflux, i.e. pumping out of the antibiotic across the cell surface. Several approaches have been developed to overcome this problem, such as for example modification of naturally occurring antibiotics in order to enhance their effectiveness or the identification of new targeting sites.

The cytoplasmic membrane provides an advantageous target site for antibiotics as it forms a defined structure composed of different lipids, wherein said structure is generally not prone to major modifications that might allow a microorganism to adapt its membrane composition in order to develop an antibiotic resistance. In order to avoid side effects when used in eukaryotes, antibiotics targeting the cytoplasmic membrane need to be selective for their respective prokaryotic target microorganisms. Antibiotics that incorporate also into eukaryotic membranes, such as for example gramicidin, are unsuitable for other than topical treatment of eukaryotes. Thus, research investigations are focusing on selective peptide antibiotics capable of assembling in the membranes of prokaryotes without affecting the membranes of eukaryotic cells, e.g. erythrocytes.

Antibiotics that target the cytoplasmic membrane may act by forming channels or pores which subsequently leads to leakage of the cells and cell death. Accordingly, such antibiotics are also referred to as channel forming peptide antibiotics or ionophores. Such substances, which are usually cationic peptides also comprising a number of hydrophobic amino acids, are generally produced by nearly every eukaryotic organism, as well as some microorganisms, to fight pathogens. These cationic peptides have a surplus of basic amino acids which leads to a positive charge in the neutral pH range. This feature is believed to be the main factor responsible for their effect (Zasloff, 2002).

Research on antimicrobial peptides targeting the cytoplasmic membrane is aided by the fact that the cytoplasmic membrane of microorganisms, and particularly of prokaryotes, differs considerably from that of eukaryotes. A major difference is regarded as the different composition of bacterial and eukaryotic membranes. Generally, bacterial membranes consist only of anionic lipids (Ingram, 1977; Clejan et al., 1986). In comparison, the external part of an erythrocyte, taken as a prominent representative of a mammalian cell, is neutral due to the presence of zwitterionic phosphatidycholines, phosphatidylamines and sphingomyelins (Verkleijet al., 1973; Casu et al., 1968). This promotes an electrostatic interaction of most cationic peptides with the bacterial membrane. The higher affinity leads to the accumulation of the peptides on the target membrane so that the limiting concentration necessary for the permeabilizing effect is reached more rapidly than on eukaryotic membranes.

The bacterial membrane is the site of oxidative phosphorylation. This requires a strong potential, with the cell interior as the negative pole. This increases the above-mentioned electrostatic interaction and accordingly, the selectivity (Hancock and Lehrer, 1998). A further difference is the presence of cholesterol in the plasma membranes of higher organisms resulting in different mechanical properties of the membrane which are discussed as another reason for the decreased binding of amphipatic molecules to these membranes (Benachir et al., 1997; Allende and McIntosh, 2003).

In addition to the cytoplasmic membrane, Gram-negative bacteria comprise an outer membrane rich in lipopolysaccharides. It is well known that some antimicrobial peptides bind to lipopolysaccharides, in particular to lipid A, and thereby permeabilize or destroy the outer membrane (Falla et al., 1996; Piers et al., 1994; Piers and Hancock, 1994). This process is also referred to as "self-promoted uptake" and enables the peptides to advance to the plasma membrane of these bacteria.

Surprisingly, so far only very few resistances are known against such antimicrobial peptides, which are, as mentioned above, part of the standard repertoire of the immune system of eukaryotic organisms. However, the efficacy of these naturally occurring antimicrobial peptides is often very low.

Due to their specificity, the side effects related to antibiotics targeting the plasma membrane of prokaryotic pathogens will most likely concern mostly non-pathogenic microorganisms such as those naturally inhabiting the skin or intestines and not the eukaryotic subjects contacted with said antibiotics.. However, such side effects may easily be compensated by resettling these non-pathogenic microorganisms after treatment.

Thus, although a number of antibiotics specific for prokaryotes is available in the art, there still exists the need for further antibiotics to overcome the drawbacks of resistances and/or low efficiency of the existing antibiotics.

Accordingly, the present invention relates to a peptide comprising or consisting of a sequence of formula I A-B-C-D-E-F (formula I), wherein A is a peptide consisting of three hydrophobic amino acid residues; B is an amino acid residue selected from the group consisting of hydrophobic and basic amino acid residues; C is a peptide consisting of two hydrophobic amino acid residues; D is a peptide consisting of two basic amino acid residues; E is a peptide consisting of two hydrophobic amino acid residues; and F is a peptide consisting of two basic amino acid residues; or a peptidomimetic thereof; wherein the basic amino acid residues are selected from the group consisting of arginine, lysine and histidine; wherein the hydrophobic amino acid residues are selected from the group consisting of leucine, alanine, valine, isoleucine, methionine and phenylalanine; wherein said peptide or peptidomimetic has antimicrobial activity.

The term "peptide" generally describes linear molecular chains of amino acids containing up to 30 amino acids covalently linked by peptide bounds, whereas the term "polypeptide", interchangeably used with the term "protein" denotes amino acid stretches of more than 30 amino acids. Peptides may form oligomers consisting of at least two identical or different molecules. The corresponding higher order structures of such multimers are, correspondingly, termed homo- or heterodimers, homo- or heterotrimers etc.. In the present invention, the sequences indicated are from the N-to the C-terminus.

The one-letter code abbreviations as used to identify amino acids throughout the present invention correspond to those commonly used for amino acids.

The peptide of the present invention can be produced synthetically. Chemical synthesis of peptides is well known in the art. Solid phase synthesis is commonly used and various commercial synthesizers are available, for example automated synthesizers by Applied Biosystems Inc., Foster City, CA; Beckman; MultiSyntech, Bochum, Germany etc. Solution phase synthetic methods may also be used, although they are less convenient. For example, peptide synthesis can be carried out using Nα-9-fluorenylmethoxycarbonyl amino acids and a preloaded trityl resin or an aminomethylated polystyrene resin with a p-carboxytritylalcohol linker. Couplings can be performed in dimethylformamide using N-hydroxybenzotriazole and 2-(1H-benzotriazole-1-yl)-1,1,3,3-tetramethyluronium hexafluorophosphate. Commonly used side chain protecting groups are tert-butyl for D, E and Y; trityl for N, Q, S and T; 2,2,4,6,7-pentamethyldihydroxybenzofruan-5-sulfonyl for R; and butyloxycarbonyl for K. After synthesis, the peptides are deprotected and cleaved from the polymer support by treatment with e.g. 92% trifluoracetic acid/ 4% triethylsilane/ 4% $H_2O$. The peptides can be precipitated by the addition of tert-butylether/pentane (8:2) and purified by reversed-phase HPLC. The peptides are commonly analysed by matrix-associated laser desorption time-of-flight mass spectrometry. By using these standard techniques, naturally occurring amino acids may be substituted with unnatural amino acids, particularly D-stereoisomers, and also with amino acids with side chains having different lengths or functionalities. Functional groups for conjugating to small molecules, label moieties, peptides, or proteins may be introduced into the molecule during chemical synthesis. In addition, small molecules and label moieties may be attached during the synthetic process. Preferably, introduction of the functional groups and conjugation to other molecules minimally affect the structure and function of the subject peptide.

The N-and C-terminus may be derivatized using conventional chemical synthetic methods. The peptides of the invention may contain an acyl group, such as an acetyl group. Methods for acylating, and specifically for acetylating the free amino group at the N-terminus are well known in the art. For the C-terminus, the carboxyl group may be modified by esterification with alcohols or amidated to form-$CONH_2$ or CONHR. Methods of esterification and amidation are well known in the art.

Furthermore, the peptide of the invention may also be produced semi-synthetically, for example by a combination of recombinant and synthetic production. In the case that fragments of the peptide are produced synthetically, the remaining part of the peptide would have to be produced otherwise, e.g. recombinantly as described further below, and then be linked to the fragment to form the peptide of the invention.

Furthermore, the invention encompasses peptidomimetics of the peptide as defined above. A peptidomimetic is a small protein- or peptide-like chain designed to mimic a peptide. Peptidomimetics typically arise from modifications of an existing peptide in order to alter the properties of the peptide. For example, they may arise from modifications to change the stability of the peptide. These modifications involve changes to the peptide that will not occur naturally (such as altered backbones and the incorporation of non-natural amino acids), including the replacement of amino acids or peptide bonds by functional analogues. Such functional analogues include all known amino acids other than the 20 gene-encoded amino acids, such as for example selenocysteine. The use of peptidomimetics as compared to other mimetics has some particular advantages. For instance, their conformationally restrained structure allows to minimize binding to non-target compounds and to enhance the activity at the desired targets. Through the addition of hydrophobic residues and/or replacement of amide bonds the transport of peptidomimetics through cellular membranes can be improved. Furthermore peptidomimetics such as isosters, retro-inverso (all-d retro or retroenantio) peptides and cyclic peptides are less susceptible to degradation by peptidases and other enzymes. Retro-inverso modification of naturally occurring peptides involves the synthetic assemblage of amino acids with $\alpha$-carbon stereochemistry opposite to that of the corresponding L-amino acids, i.e. D- or D-allo-amino acids, in reverse order with respect to the native peptide sequence. A retro-inverso analogue thus has reversed termini and reversed direction of peptide bonds while approximately maintaining the topology of the side chains as in the native peptide sequence.

"Basic amino acid residues" in accordance with the present invention are amino acid residues that are polar and positively charged at pH values below their $pK_a$'s, and that are very hydrophilic. Basic amino acid residues are arginine, lysine and histidine. Arginine, an essential amino acid, has a positively charged guanidino group. Arginine is well designed to bind the phosphate anion, and is often found in the active centres of proteins that bind phosphorylated substrates. Lysine, another essential amino acid, has a positively charged $\varepsilon$-amino group (a primary amine). Lysine is basically alanine with a propylamine substituent on the $\beta$-carbon. The $\varepsilon$-amino group has a significantly higher $pK_a$ (about 10.5 in polypeptides) than does the $\alpha$-amino group. The amino group is highly reactive and often participates in reactions at the active centres of enzymes. Proteins only have one $\alpha$ amino group, but numerous $\varepsilon$ amino groups. However, the higher $pK_a$ renders the lysyl side chains effectively less nucleophilic. As cations, arginine as well as lysine play a role in maintaining the overall charge balance of a protein. Histidine, an essential amino acid, has as a positively charged imidazole functional group.

The group of hydrophobic amino acids according to the invention contains nonpolar amino acids and is comprised of aliphatic and aromatic amino acids as well as methionine. In case of aliphatic amino acids, hydrophobicity increases with increasing number of C atoms in the hydrocarbon chain. Although these amino acids prefer to remain inside protein molecules, alanine and glycine are ambivalent, meaning that they can be inside or outside the protein molecule. Glycine has such a small side chain that it does not have much effect on the hydrophobic interactions. The hydrophobicity increases from glycine to alanine, valine, leucine and isoleucine. The group of aromatic amino acids composed of tyrosine, tryptophane and phenylalanine is also characterized by the hydrophobicity which increases from tyrosine to tryptophane and phenylalanine as the most hydrophobic aromatic amino acid. Due to its hydrophobicity, phenylalanine is nearly always found buried within a protein. The $\pi$ electrons of the phenyl ring can stack with other aromatic systems and often do within folded proteins, adding to the stability of the structure. Methionine, an essential amino acid, is one of the two sulfur-containing amino acids. The side chain is quite hydrophobic and methionine is usually found buried within proteins. Unlike cysteine, the sulfur of methionine is not highly nucleophilic, although it will react with some electrophilic centers.

The term "antimicrobial activity" in accordance with the present invention refers to the killing of microorganisms or fungi or the prevention of the growth of microorganisms or fungi by the peptide of the invention. The skilled person knows means and methods to determine whether a peptide has an antimicrobial activity, including but not limited to microdilution assays (Schwab et al. 1999) or as shown in the Examples.

The peptide or peptidomimetic of the invention preferably integrates into a cytoplasma membrane, preferably a cytoplasma membrane of a microorganism, in particular of prokaryotic cells, leading either to membrane destruction or to the formation of a channel (interchangeably used herein with the term "pore"). These features are believed to be responsible for the antimicrobial activity of the peptides and peptidomimetics of the invention, leading to cell death of the targeted microorganism. The exact process of channel formation is so far not known. In this regard, it is envisaged in the present invention that the molecules of the invention may either form channels or may alter the activity of existing channels and membrane proteins by binding to these channels or proteins. Methods for the analysis of channel formation and membrane destruction are well known to the person skilled in the art and include, but are not limited to the use of artificial membrane systems such as described in Cabrera *et al*. 2008 or Pate and Blazyk 2008.

Preferably, the peptide or peptidomimetic of the invention is a channel-forming antimicrobial peptide or peptidomimetic.

In accordance with the present invention, a group of peptides and peptidomimetics is provided that are efficient as antibiotics. More particularly, the peptides and peptidomimetics of the present invention show antimicrobial activity towards prokaryotes without an apparent effect on eukaryotic cells. More specifically, they are active in pore forming in prokaryotic cytoplasmic membranes without acting on eukaryotic cytoplasmic membranes, for example of erythrocytes. Since, as described above, the membrane structure of prokaryotes is not prone to major modifications, a resistance of prokaryotes to the peptides and peptidomimetics of the present invention is not to be expected. Consequently, the peptides and peptidomimetics of the invention are particularly suitable for targeting microorganisms, in particular pathogenic bacteria, while not having any noteworthy haemolytic activity.

Without wishing to be bound by any theory it is assumed that the structural motif of the peptides according to the present invention is responsible for these characteristics. The predicted secondary structure of the peptides is α-helical (secondary structure prediction was carried out using the program NNPREDICT available at http://www.cmpharm.ucsf.edu/~nomi/nnpredict.html), a structural motif common to many peptides incorporating into biological membranes. Furthermore, the hydrophobicity index was calculated for the hydrophobic areas of the present peptides which was found to lie in the range of between 2.06 and 3.65. A certain degree o hydrophobicity is a prerequisite for incorporating into lipid membranes.

More specifically, it is assumed that the particular assembly of basic amino acid residue stretches and their arrangement relative to hydrophobic amino acid residues in the peptides of the invention provides for the observed characteristics. More specifically, the antimicrobial peptides are amphipathic molecules containing clusters of amino acids with hydrophobic and positively charged side chains. These features allow them to interact with negatively charged as well as hydrophobic compounds of membranes. As a consequence of the interaction the peptides incorporate into the membranes resulting in pore formation or membrane destruction. In particular, the peptides display a preference for the more anionic membranes of prokaryotes rather than the neutral membranes of e.g. erythrocytes. In addition, the antimicrobial peptides of the present invention display a broad spectrum of activity including, for example, activity against plant and human pathogens.

In a preferred embodiment, the amino acid residues of the peptide of A are selected from the group consisting of leucine, alanine and isoleucine.

In a preferred embodiment, B is selected from the group consisting of alanine, arginine, lysine and histidine.

In a preferred embodiment, the amino acid residues of the peptide of C are selected from the group consisting of phenylalanine, leucine and alanine.

In a preferred embodiment, the amino acid residues of the peptide of D are selected from the group consisting of arginine and lysine, more preferably the first amino acid residue is lysine and the second amino acid residue is selected from the group consisting of lysine and arginine.

In a preferred embodiment, the amino acid residues of the peptide of E are selected from the group consisting of leucine, alanine, phenylalanine, isoleucine and valine.

In a preferred embodiment, the amino acid residues of the peptide of F are selected from the group consisting of lysine and histidine.

In a preferred embodiment, the peptide comprises or consists of the following amino acid sequence: A1-A2-A3-A4-A5-A6-A7-A8-A9-A10-A11-A12 (formula II), wherein A1, A2, A3, A5, A6, A9, A10 are hydrophobic amino acid residues; A4 is an amino acid residue selected from the group consisting of hydrophobic and basic amino acid residues; A7, A8, A11,A12 are basic amino acid residues.

In a preferred embodiment, A1 is selected from the group consisting of alanine and leucine.

In a preferred embodiment, A2 is leucine.

In a preferred embodiment, A3 is selected from the group consisting of isoleucine and alanine.

A1 to A3 form part A of the peptide of the invention.

In a preferred embodiment, A4 is selected from the group consisting of alanine, arginine, lysine and histidine. A4 forms part B of the peptide of the invention.

In a preferred embodiment, A5 is selected from the group consisting of alanine and phenylalanine.

In a preferred embodiment, A6 is selected from the group consisting of alanine, leucine and phenylalanine.

A5 and A6 form part C of the peptide of the invention.

In a preferred embodiment, A7 is lysine.

In a preferred embodiment, A8 is selected from the group consisting of arginine and lysine.

A7 and A8 form part D of the peptide of the invention.

In a preferred embodiment, A9 is selected from the group consisting of alanine, leucine and phenylalanine.

In a preferred embodiment, A10 is selected from the group consisting of valine and isoleucine.

A9 and A10 form part E of the peptide of the invention.

In a preferred embodiment, A11 is lysine.

In a preferred embodiment, A12 is selected from the group consisting of lysine and histidine.

A11 and A12 form part F of the peptide of the invention.

In a preferred embodiment, the peptide comprises or consists of a sequence selected from the group consisting of LLIAAFKKLVKK (SP5; SEQ ID NO: **1**), ALAHFLKKAIKK (SP6; SEQ ID NO: **2**), LLIKFLKRFIKH (SP7; SEQ ID NO: 3) and LLIRAAKKFIKK (SP8; SEQ ID NO: **4**).

As shown in the appended examples, peptides of the invention comprising an amino acid sequence as outlined above, have antimicrobial activity. Said peptides include, but are not limited to, peptides consisting of the respective sequence but also having additional amino acid residues at either their N-terminus, their C-terminus or at both ends, preferably while essentially retaining their antimicrobial activity. Such additional amino acid residues, also referred to as flanking regions, at the termini of the peptide of the invention are preferably at least one amino acid residue, more preferably at least two amino acid residues, more preferably at least three amino acid residues and even more preferably at least four amino acid residues, such as at least five, six or seven amino acid residues.

In a preferred embodiment, the amino acid residues of the peptide of the invention are L-amino acid residues.

In another preferred embodiment, the peptide comprises one or several D-amino acids. From results obtained with known antimicrobial peptides forming pores/channels, it is assumed that the peptides of the present invention act on non-chiral structures of plasma membranes. Accordingly, peptides comprising one or more, such as at least two, at least three, at least four, at least five, at least 10 or 12 D-amino acids will exert the same antimicrobial effect as peptides consisting of L-amino acids.

In another preferred embodiment, the peptide comprises or consists of the sequence LL*I*KF***LKR***F*I*KH, wherein the amino acid residues represented in bold and italics represent D-amino acid residues. As apparent from the appended examples, said peptide shows antimicrobial activity.

In a further preferred embodiment, all amino acids of the peptide are D-amino acids.

In a more preferred embodiment, the antimicrobial activity of the peptide or peptidomimetic is directed against Gram-negative and/or Gram-positive bacteria. The peptides of the present invention exert their antimicrobial activity against both classes of bacteria despite their differences in membrane structure and function.

The antimicrobial activity of the peptides and peptidomimetics of the present invention can be compared to that of protegrin and/or magainin which are both pore-forming antimicrobials.

Protegrin-I (PG-1) is composed of 18 amino acids with a high content of cysteine and positively charged amino arginine residues. The peptide displays an antimicrobial activity (Kokryakov *et al.*, 1993) by forming a pore/channel that leads to cell death (Panchal *et al.*, 2002; Sokolov *et al.*, 1999). Unlike most other antimicrobial peptides forming α-helical structures, PG-1 adopts a β-sheet motif (Fahmer *et al.*, 1996).

Magainins (23 amino acids) constitute a family of peptides isolated from the skin of the African clawed frog Xenopus laevis. Magainin-1 and Magainin-2 are closely related peptides of 23 amino acids that differ by two substitutions (Zasloff, 1987). Both peptides are pore-forming peptides (Matsuzaki, 1999), non hemolytic, and inhibit growth of numerous species of bacteria and fungi and induce osmotic lysis of protozoa (Zasloff, 1987; Cruciani *et al.*, 1991). Kuzina et al (2006) have reported that Magainin-2 is active against the plant pathogenic bacterium Xylella fastidiosa. It is particularly preferred that Maginin-2 is used when a comparison of the activity of the peptides of the present invention with Magainins is performed.

The antimicrobial activity of the above peptides has been extensively examined and these peptides can be obtained commercially, so that they are suitable for comparison with the peptides of the present invention. Comparison of antimicrobial activity may conveniently be carried out by determining the minimum inhibitory concentration (MIC) of a peptide of the invention and comparing the observed MIC with data obtained for protegrin and/or magainin.

Further suitable peptides for comparison with the peptides of the present invention are histatin 5 and cathepsin.

Histatins are 3- to 4-kDa structurally related histidine-rich basic proteins produced only in humans and higher subhuman primates. Histatin 5 is a salivary peptide of 24 amino acids that targets fungal mitochondria. It is the most potent candidacidal member of the histatin-family *in vitro,* killing yeast and filamentous forms of Candida species at physiological concentrations (from 15 to 30 μM).

Lysosomal cathepsin G from human neutrophils is a chymotrypsin-like protease, which also possesses antimicrobial activity. The antimicrobial activity, however, is independent of protease activity, because treatment of this enzyme with the irreversible serine protease inhibitor diisopropylfluorophosphate has no effect on its antimicrobial action. Cathepsin G (77-83) was originally isolated from a clostripain digest of lysosomal cathepsin G. This peptide consists of 7 amino acids and represents the antimicrobial domain within the human neutrophil cathepsin G. It has been found to exert broad-spectrum antimicrobial activity in vitro. Depending on the target bacterial strain, these peptides exhibited antimicrobial activity between 50 μM and 400 μM.

In a preferred embodiment of the peptide of the invention, the antimicrobial activity is directed against at least one organism selected from the group of plant pathogens consisting of *Pseudomonas syringae pv. Syringae* (G-), *Pseudomonas syringae pv. Tomato* (G-), *Pseudomonas corrugate* (G-), *Pectobacterium carotovorum sub. Carotovorum* (G-), *Clavibacter michiganensis sub. Michiganensis* (G+), *Xanthomonas vesicatoria* (G-) *Erwinia amylovora (G-), Botrytis cinerea, Alternaria alternata* and *Cladosporium herbarum* and from the group of human pathogens consisting of *Enterobacter cloacae* (G-), *Yersinia enterocolitica* (G-), *Klebsiella pneumoniae* (G-), *Klebsiella oxytoca* (G-), *Pseudomonas aeruginosa* (G-), *Staphylococcus aureus* (G-) and *Staphylococcus epidermidis* (G-) and including also multi resistant strains.

The term "multi resistant strains" in accordance with the present invention refers to strains of the above mentioned pathogens that are no longer susceptible to previously effective antimicrobial drugs as they have developed resistances against these drugs. A multi resistant strain may, for example, develop due to random genetic mutations in the pathogens that alter their sensitivity to these drugs.

The peptide or peptidomimetic of the invention has a low hemolytic activity, as also shown in the appended examples. The term "low hemolytic activity" in accordance with the present invention denotes the ratio of the concentration of peptide where antimicrobial activity is observed and the concentration of peptide where haemolytic activity is observed, wherein the ratio of the two concentrations is at least 50:1 (effective antimicrobial concentration: haemolytic concentration), preferably at least 100:1, more preferably at least 200: 1 such as 500:1 or 1000:1. Most preferably, the ratio of the two concentrations is at least 2000:1.

Methods to determine the haemolytic activity are well-known in the art and are also disclosed in the appended examples.

In another embodiment, the present invention relates to a nucleic acid molecule encoding the peptide of the invention.

The term "nucleic acid molecule" as used interchangeably with the term "polynucleotide", in accordance with the present invention, includes DNA, such as cDNA or genomic DNA, and RNA. Further included are nucleic acid mimicking molecules known in the art such as synthetic or semi-synthetic derivatives of DNA or RNA and mixed polymers. Such nucleic acid mimicking molecules or nucleic acid derivatives according to the invention include phosphorothioate nucleic acid, phosphoramidate nucleic acid, 2'-O-methoxyethyl ribonucleic acid, morpholino nucleic acid, hexitol nucleic acid (HNA), peptide nucleic acid (PNA) and locked nucleic acid (LNA) (see Braasch and Corey, Chem Biol 2001, 8: 1). LNA is an RNA derivative in which the ribose ring is constrained by a methylene linkage between the 2'-oxygen and the 4'-carbon. They may contain additional non-natural or derivative nucleotide bases, as will be readily appreciated by those skilled in the art.

In a preferred embodiment, the nucleic acid molecule is DNA.

It will be readily appreciated by the skilled person that more than one nucleic acids may encode the peptide of the present invention due to the degeneracy of the genetic code. Degeneracy results because a triplet code designates 20 amino acids and a stop codon. Because four bases exist which are utilized to encode genetic information, triplet codons are required to produce at least 21 different codes. The possible $4^3$ possibilities for bases in triplets give 64 possible codons, meaning that some degeneracy must exist. As a result, some amino acids are encoded by more than one triplet, i.e. by up to six. The degeneracy mostly arises from alterations in the third position in a triplet. This means that nucleic acid molecules having a different sequences, but still encoding the same polypeptide lie within the scope of the present invention.

Further, the invention relates to a vector comprising the nucleic acid molecule of the invention.

Preferably, the vector is a plasmid, cosmid, virus, bacteriophage or another vector used conventionally e.g. in genetic engineering.

Preferably, the vector is an expression vector.

An expression vector according to this invention is capable of directing the replication, and the expression of the nucleic acid molecule of the invention and the peptide, fusion peptide or fusion polypeptide encoded thereby. Suitable expression vector are described above.

The nucleic acid molecule of the present invention may be inserted into several commercially available vectors. Non-limiting examples include prokaryotic plasmid vectors, such as the pUC-series, pBluescript (Stratagene), the pET-series of expression vectors (Novagen) or pCRTOPO (Invitrogen), lambda gt11, pJOE, the pBBR1-MCS series, pJB861, pBSMuL, pBC2, pUCPKS, pTACT1 and vectors compatible with expression in mammalian cells like pREP (Invitrogen), pCEP4 (Invitrogen), pMC1neo (Stratagene), pXT1 (Stratagene), pSG5 (Stratagene), EBO-pSV2neo, pBPV-1, pdBPVMMTneo, pRSVgpt, pRSVneo, pSV2-dhfr, pIZD35, Okayama-Berg cDNA expression vector pcDV1 (Pharmacia), pRc/CMV, pcDNA1, pcDNA3 (Invitrogene), pSPORT1 (GIBCO BRL), pGEMHE (Promega), pLXIN, pSIR (Clontech), pIRES-EGFP (Clontech), pEAK-10 (Edge Biosystems) pTriEx-Hygro (Novagen) and pCINeo (Promega). Examples for plasmid vectors suitable for Pichia pastoris comprise e.g. the plasmids pAO815, pPIC9K and pPIC3.5K (all Invitrogen).

The nucleic acid molecule of the present invention referred to above may also be inserted into vectors such that a translational fusion with another nucleic acid molecule is generated. The other nucleic acid molecules may encode a protein which may e.g. increase the solubility and/or facilitate the purification of the protein encoded by the nucleic acid molecule of the invention. Non-limiting examples include pET32, pET41, pET43. Furthermore, the other nucleic acid molecule may encode a peptide or protein which enables for the compensation of the toxic properties of the antimicrobial peptides of the invention which would otherwise harm or kill the host cell (see below).

The vectors may also contain an additional expressible polynucleotide coding for one or more chaperones to facilitate correct protein folding. Suitable bacterial expression hosts comprise e. g. strains derived from BL21 (such as BL21(DE3), BL21(DE3)PlysS, BL21(DE3)RIL, BL21(DE3)PRARE) or Rosetta®.

For vector modification techniques, see Sambrook and Russel (2001). Generally, vectors can contain one or more origins of replication (ori) and inheritance systems for cloning or expression, one or more markers for selection in the host, e.g., antibiotic resistance, and one or more expression cassettes. Suitable origins of replication include, for example, the Col E1, the SV40 viral and the M 13 origins of replication.

The coding sequences inserted in the vector can e.g. be synthesized by standard methods, or isolated from natural sources. Ligation of the coding sequences to transcriptional regulatory elements and/or to other amino acid encoding sequences can be carried out using established methods. Transcriptional regulatory elements (parts of an expression cassette) ensuring expression in prokaryotes or eukaryotic cells are well known to those skilled in the art. These elements comprise regulatory sequences ensuring the initiation of the transcription (e. g., translation initiation codon, promoters, enhancers, and/or insulators), internal ribosomal entry sites (IRES) (Owens *et al*., 2001) and optionally poly-A signals ensuring termination of transcription and stabilization of the transcript. Additional regulatory elements may include transcriptional as well as translational enhancers, and/or naturally-associated or heterologous promoter regions. Preferably, the nucleic acid molecule of the invention is operably linked to such expression control sequences allowing expression in prokaryotes or eukaryotic cells.

A typical mammalian expression vector contains the promoter element, which mediates the initiation of transcription of mRNA, the protein coding sequence, and signals required for the termination of transcription and polyadenylation of the transcript. Moreover, elements such as origin of replication, drug resistance gene, regulators (as part of an inducible promoter) may also be included. The *lac* promoter is a typical inducible promoter, useful for prokaryotic cells, which can be induced using the lactose analogue *isopropylthiol-b-D-galactoside.* ("IPTG"). For recombinant expression, the antibody fragment may be ligated between e.g. the PelB leader signal, which directs the recombinant protein in the periplasm and the gene III in a phagemid called pHEN4 (described in Ghahroudi *et al*, 1997). Additional elements might include enhancers, Kozak sequences and intervening sequences flanked by donor and acceptor sites for RNA splicing. Highly efficient transcription can be achieved with the early and late promoters from SV40, the long terminal repeats (LTRs) from retroviruses, e.g., RSV, HTLVI, HIVI, and the early promoter of the cytomegalovirus (CMV). However, cellular elements can also be used (e.g., the human actin promoter). The co-transfection with a selectable marker such as dhfr, gpt, neomycin, hygromycin allows the identification and isolation of the transfected cells. The transfected nucleic acid can also be amplified to express large amounts of the encoded (poly)peptide. The DHFR (dihydrofolate reductase) marker is useful to develop cell lines that carry several hundred or even several thousand copies of the gene of interest. Another useful selection marker is the enzyme glutamine synthase (GS) (Murphy et al. 1991; Bebbington et al. 1992). Using these markers, the mammalian cells are grown in selective medium and the cells with the highest resistance are selected. As indicated above, the expression vectors will preferably include at least one selectable marker. Such markers include dihydrofolate reductase, G418 or neomycin resistance for eukaryotic cell culture and tetracycline, kanamycin or ampicillin resistance genes for culturing in *E. coli* and other bacteria.

Possible regulatory elements permitting expression in prokaryotic host cells comprise, e.g., the *lac, trp* or tac promoter, the lacUV5 or the trp promotor in *E. coli,* and examples for regulatory elements permitting expression in eukaryotic host cells (the more preferred embodiment) are the *AOX1* or *GAL1* promoter in yeast or the CMV-(Cytomegalovirus), SV40-, RSV-promoter (Rous sarcoma virus), chicken beta-actin promoter, CAG-promoter (a combination of chicken beta-actin promoter and cytomegalovirus immediate-early enhancer), the gai10 promoter, human elongation factor 1α-promoter, CMV enhancer, CaM-kinase promoter, the Autographa californica multiple nuclear polyhedrosis virus (AcMNPV) polyhedral promoter or a globin intron in mammalian and other animal cells. Besides elements which are responsible for the initiation of transcription such regulatory elements may also comprise transcription termination signals, such as the SV40-poly-A site or the tk-poly-A site or the SV40, lacZ and AcMNPV polyhedral polyadenylation signals, downstream of the polynucleotide.

The vector may further comprise nucleotide sequences encoding signal peptides or further regulatory elements. Such sequences are well known to the person skilled in the art. Furthermore, depending on the expression system used, leader sequences capable of directing the expressed polypeptide to a cellular compartment may be added to the coding sequence of the nucleic acid molecule of the invention. Such leader sequences are well known in the art. Specifically-designed vectors allow the shuttling of DNA between different hosts, such as bacteria-fungal cells or bacteria-animal cells. The nucleic acid molecules of the invention as described herein above may be designed for direct introduction or for introduction via liposomes, phage vectors or viral vectors (e.g. adenoviral, retroviral) into the cell. Additionally, baculoviral systems or systems based on Vaccinia Virus or Semliki Forest Virus can be used as vector in eukaryotic expression system for the nucleic acid molecules of the invention. Expression vectors derived from viruses such as retroviruses, vaccinia virus, adeno-associated virus, herpes viruses, or bovine papilloma virus, may be used for delivery of the polynucleotides or vector into targeted cell population. Methods which are well known to those skilled in the art can be used to construct recombinant viral vectors; see, for example, the techniques described in Sambrook, 2001 and Ausubel, 2001.

The invention furthermore refers to a host cell comprising the nucleic acid molecule or the vector of the invention. In a preferred embodiment, the host cell is a microorganism, preferably a unicellular microorganism.

Suitable prokaryotic host cells comprise e.g. bacteria of the genera Escherichia, Streptomyces, Salmonella or Bacillus. It is of note that in case prokaryotic host cells are used, the vector of the invention preferably comprises the fusion peptide or fusion polypeptide of the invention if the expressed peptide alone would be toxic to said prokaryotic cells.

Suitable eukaryotic host cells are e.g. yeasts such as Saccharomyces cerevisiae or Pichia pastoris. Insect cells suitable for expression are e.g. Drosophila S2 or Spodoptera Sf9 cells. In order to be able to express the peptide of the invention in sufficient amounts, preferably the fusion peptide or the fusion polypeptide is encoded by the vector of the present invention if the expression of the peptide of the invention alone would be toxic to the host cell. This can easily be determined by the skilled person using routine biotechnological methods such as a test expression. Mammalian host cells that could be used include, human Hela, HEK293, H9 and Jurkat cells, mouse NIH3T3 and C127 cells, COS 1, COS 7 and CV1, quail QC1-3 cells, mouse L cells, Bowes melanoma cells and Chinese hamster ovary (CHO), cells. Also within the scope of the present invention are primary mammalian cells or cell lines. Primary cells are cells which are directly obtained from an organism. Suitable primary cells are, for example, mouse embryonic fibroblasts (MEF), mouse primary hepatocytes, cardiomyocytes and neuronal cells as well as mouse muscle stem cells (satellite cells) and stable, immortalized cell lines derived thereof. Alternatively, the recombinant protein of the invention can be expressed in stable cell lines that contain the gene construct integrated into a chromosome.

Appropriate culture media and conditions for the above-described host cells are known in the art.

Alternatively, the host cell is an isolated cell. Such a cell has been isolated from a tissue of a multicellular organism or forms a protozoon. In the case of eukaryotic cells, the host cell of the present invention has been separated from the tissue where it is normally found. Cells found in cell culture, preferably in liquid cell culture, are isolated cells in accordance with the present invention.

In another preferred embodiment, the host cell is a plant cell.

Suitable plant cells are e.g. those belonging to species infectable by plant pathogens sensitive to the peptides of the present invention. Examples of such plant pathogens are given further above. They infect e.g. Syringa or Phaseolus species, Lycopersicon (tomato), carrots, solanum (potatoe), pomoidae of the rosaceae such as apple, pear or raspberry. Plants or plant cells suitable as host cells and infectable by said plant pathogens can be transformed with an expression vector of the invention as protective agent. The production of transgenic plants expressing the peptide of the invention is well known to the person skilled in the art (Aerts *et al.*, 2007; Oard and Enright, 2006). Such transgenic plants or parts thereof may be infected with bacteria, fungi or viruses and analysed for the protective effect of the peptide as compared to control experiments in wild-type plants. Alternatively, plant cells or plants can simply serve for production purposes.

In a different embodiment, the invention relates to a method for producing a peptide of the invention, comprising culturing the host of the invention under suitable conditions and isolating the peptide produced.

A large number of suitable methods exist in the art to produce peptides in appropriate hosts. If the host is a unicellular organism such as a prokaryote or a mammalian or insect cell, the person skilled in the art can revert to a variety of culture conditions. Conveniently, the produced protein is harvested from the culture medium, lysates of the cultured cells or from isolated (biological) membranes by established techniques. A preferred method involves the synthesis of nucleic acid sequences by PCR and its insertion into an expression vector. Subsequently a suitable host cell may be transfected or transformed etc. with the expression vector. Thereafter, the host cell is cultured to produce the desired peptide, which is isolated and purified.

Appropriate culture media and conditions for the above-described host cells are known in the art. For example, suitable conditions for culturing bacteria are growing them under aeration in Luria Bertani (LB) medium. To increase the yield and the solubility of the expression product, the medium can be buffered or supplemented with suitable additives known to enhance or facilitate both. E. coli can be cultured from 4 to about 37 °C, the exact temperature or sequence of temperatures depends on the molecule to be overexpressed. In general, the skilled person is also aware that these conditions may have to be adapted to the needs of the host and the requirements of the peptide or protein expressed. In case an inducible promoter controls the nucleic acid of the invention in the vector present in the host cell, expression of the polypeptide can be induced by addition of an appropriate inducing agent. Suitable expression protocols and strategies are known to the skilled person.

Depending on the cell type and its specific requirements, mammalian cell culture can e.g. be carried out in RPMI or DMEM medium containing 10% (v/v) FCS, 2mM L-glutamine and 100 U/ml penicillin/streptomycin. The cells can be kept at 37 °C in a 5% $CO_2$, water saturated atmosphere. Suitable media for insect cell culture is e.g. TNM + 10% FCS or SF900 medium. Insect cells are usually grown at 27 °C as adhesion or suspension culture.

Suitable expression protocols for eukaryotic cells are well known to the skilled person and can be retrieved e.g. from Sambrook, 2001.

As described above, when producing the peptide of the invention in a host cell, the expression vector preferably encodes a fusion peptide or fusion polypeptide if the peptide produced exerts toxic activity towards the host cell selected. Furthermore, the vector may encode a fusion peptide or polypeptide, wherein the peptide of the invention is coupled to a signal peptide to direct expression to a specific compartment or site or to a tag which facilitates purification of the fusion peptide or polypeptide. Suitable tags are well known in the art and comprise e.g. a hexahistidine tag and a GST (glutathione S-transferase) tag.

The fusion peptide or fusion polypeptide expressed has to be processed in order to cleave the compensating but undesired peptide or polypeptide or the signal peptide or tag fused to the peptide of the invention. This can take place at any stage of the purification process after culturing the host cell. Suitable methods to cleave off the undesired part are either chemical methods using e.g. cyanogen bromide which cleaves at methionine residues or N-chloro succinimide which cleaves at tryptophan residues. Alternatively, enzymatic methods can be used which are in general more gentle than chemical methods. Exemplary proteases suitable for cleavage are specific for a certain amino acid sequence and include Factor Xa or TEV protease.

An alternative method for producing the peptide of the invention is in vitro translation of mRNA. Suitable cell-free expression systems for use in accordance with the present invention include rabbit reticulocyte lysate, wheat germ extract, canine pancreatic microsomal membranes, *E. coli* S30 extract, and coupled transcription/translation systems such as the TNT-system (Promega). These systems allow the expression of recombinant peptides or proteins upon the addition of cloning vectors, DNA fragments, or RNA sequences containing coding regions and appropriate promoter elements.

Methods of isolation of the peptide produced are well-known in the art and comprise, without limitation, method steps such as ion exchange chromatography, gel filtration chromatography (size exclusion chromatography), affinity chromatography, high pressure liquid chromatography (HPLC), reversed phase HPLC, disc gel electrophoresis or immunoprecipitation (see, for example, Sambrook, 2001).

In a different embodiment, the present invention relates to a composition comprising a peptide or peptidomimetic of the invention, the nucleic acid molecule of the invention, the vector of the invention or the host cell of the invention.

In a preferred embodiment, the composition is selected from the group consisting of a pharmaceutical composition or a plant protective composition; and optionally further comprises a suitable carrier and/or diluent.

In accordance with the present invention, the term "pharmaceutical composition" relates to a composition for administration to a patient, preferably a human patient. The pharmaceutical composition of the invention comprises the peptide of the invention. It may, optionally, comprise further molecules capable of altering the characteristics of the peptide of the invention thereby, for example, stabilizing, modulating and/or activating its function. The composition may be in solid, liquid or gaseous form and may be, inter alia, in the form of (a) powder(s), (a) tablet(s), (a) solution(s) or (an) aerosol(s). The pharmaceutical composition of the present invention may, optionally and additionally, comprise a pharmaceutically acceptable carrier. By "suitable carrier" interchangeably used in connection with the pharmaceutical composition of the present invention with "pharmaceutically acceptable carrier" is meant a non-toxic solid, semisolid or liquid filler, diluent, encapsulating material or formulation auxiliary of any type. Examples of suitable pharmaceutical carriers are well known in the art and include phosphate buffered saline solutions, water, emulsions, such as oil/water emulsions, various types of wetting agents, sterile solutions, organic solvents including DMSO etc. Pharmaceutical carriers are chosen according to the desired mode of administration. Compositions comprising such carriers can be formulated by well known conventional methods. These pharmaceutical compositions can be administered to the subject at a suitable dose. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. The therapeutically effective amount for a given situation will readily be determined by routine experimentation and is within the skills and judgement of the ordinary clinician or physician. Generally, the regimen as a regular administration of the pharmaceutical composition should be in the range of 1 µg to 5 g units per day. However, a more preferred dosage might be in the range of 0.01 mg to 100 mg, even more preferably 0.01 mg to 50 mg and most preferably 0.01 mg to 10 mg per day.

The pharmaceutical composition of the present invention may be administered e.g. systemically, topically or parenterally. The term "parenteral" as used herein refers to modes of administration which include intravenous, intramuscular, intraperitoneal, intrasternal, subcutaneous and intraarticular injection and infusion.

The term "plant-protective composition" relates to compositions used in the prevention or treatment of diseases related to plants. Formulations of plant-protective compositions comprise wettable powders (WPs) and emulsifiable concentrates (ECs). Being liquids, the EC compositions are easier to handle, their portioning can be handled by a simple volumetric measurement. The biological activity of the EC compositions is usually higher than that of the WP compositions. EC compositions can be prepared only from an active ingredient which is liquid or, when a solvent can be found in which the active ingredient can be dissolved to give a solution of 10 to 85% concentration (depending on the usual concentrations of the application) without any risk of the interim alteration of the active ingredient. EC compositions usually contain a high amount of solvent. The drawback of the EC compositions can be diminished by formulating the active ingredient in an emulsifiable microemulsion concentrate. Microemulsion is a colloidal system which, in a first approach differs from a true emulsion in the dimension of its particles which are smaller by an order of magnitude than those of a true emulsion. According to the general definition, this system contains surface active agents and two immiscible liquids, one of them is usually water, though, in principle, it is also possible to prepare a water-free microemulsion by using another solvent.

The surfactant may be the mixture of even 6 to 8 tensides and additionally, it may contain alcohols or amines of medium chain length as auxiliary surfactants (co-surfactants). A "suitable carrier" in connection with the plant protective composition is a non-toxic solid, semisolid or liquid filler, diluent, encapsulating material or formulation auxiliary of any type. Examples of suitable carriers are well known in the art and comprise water or organic solvents as liquids. The suitable composition comprising the peptides of the invention as plant protective composition can be readily determined by the skilled person using methods well-known in the art. Non limiting examples of such methods are the treatment of plants or plant tissues with compositions comprising the peptide of interest and the subsequent infection with bacteria, fungi or viruses and analysing the protective effect of the peptide as compared to control experiments in un-protected plants and plant tissues (see for example Alan and Earle, 2002).

In another embodiment, the present invention relates to the peptide or peptidomimetic of the invention, the nucleic acid molecule of the invention, the vector of the invention or the host cell the invention for use in treating infectious diseases.

An infectious disease according to the present invention is a clinically evident disease resulting from the presence of pathogenic microbial agents, including bacteria. These pathogens are able to cause disease in animals and/or plants.

Among the almost infinite varieties of microorganisms, relatively few cause disease in otherwise healthy individuals. Infectious disease results from the interplay between those few pathogens and the defenses of the hosts they infect. The appearance and severity of disease resulting from any pathogen depends upon the ability of that pathogen to damage the host as well as the ability of the host to resist the pathogen. Infectious microorganisms, or microbes, are therefore classified as either *primary pathogens* or as *opportunistic pathogens* according to the status of host defenses. Primary pathogens cause disease as a result of their presence or activity within the normal, healthy host, and their intrinsic virulence (the severity of the disease they cause) is, in part, a necessary consequence of their need to reproduce and spread. Many of the most common primary pathogens of humans only infect humans, however many serious diseases are caused by organisms acquired from the environment or which infect non-human hosts.

In a preferred embodiment, the infectious disease is an infectious disease of animals, preferably mammals and more preferably human beings.

In another preferred embodiment, the infectious disease is an infectious disease of plants.

Exemplary microorganisms causing infectious diseases in animals or plants have been described above.

In addition, the present invention relates to a kit comprising one or more peptides or peptidomimetics of the invention, one or more peptides produced by the method of the invention, the nucleic acid molecule of the invention, the expression vector of the invention or the host cell of the invention.

The various components of the kit may be packaged in one or more containers such as one or more vials. The vials may, in addition to the components, comprise preservatives or buffers for storage. Furthermore, the kit of the invention may comprise instructions for use.

The figures show:

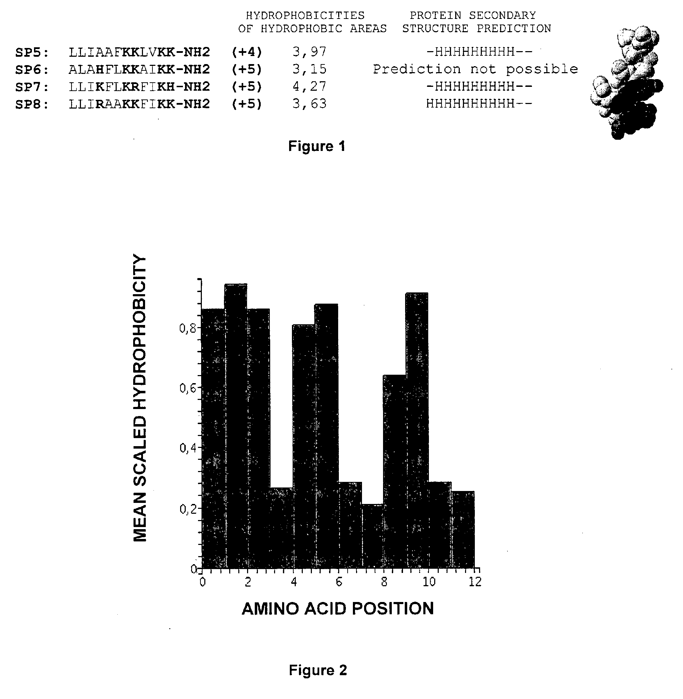

**Fig. 1** shows a model of the polypeptides of the present invention including the amino acid sequence of the preferred polypeptides, the hydrophobicities of hydrophobic areas and an indication of those amino acids and amino acid positions, respectively, which are involved in the formation of a helix as predicted by NNPREDICT and indicated by "H"; regions, where a secondary structure prediction was not possible are marked with a dash.

**Fig. 2** shows the hydrophobicity plots of the polypeptides of the invention. The graphs show the mean hydrophobicity of the peptides. The x-axis describes the positions of amino acids in the peptide; the value of the y-axis shows the mean hydrophobicity of the amino acids at the corresponding position in the peptide. The hydrophobicities given are the "Scaled" values from computational log(P) determinations by the "Small Fragment Approach" (see, "Development of Hydrophobicity Parameters to Analyze Proteins Which Bear Post- or Cotranslational Modifications" Black, S.D. and Mould, D.R. (1991) Anal. Biochem. 193, 72-82). The equation used to scale raw log(P) values to the scaled values given is as follows: Scaled Parameters = (Raw Parameters + 2.061)/4.484.

The examples illustrate the invention.

**Example 1: Synthesis of polypeptides according to the present invention**

Polypeptides were synthesized and purified (> 80% purity) from metabion (Munich, Germany). All polypeptides were amidated at their C-terminus.

Preferred polypeptides and their amino acid sequences according to the present invention are thus as follows.

SP5: LLIAAFKKLVKK (SEQ ID NO: 1)
SP6: ALAHFLKKAIKK (SEQ ID NO: 2)
SP7: LLIKFLKRFIKH (SEQ ID NO: 3) and
SP8: LLIRAAKKFIKK (SEQ ID NO: 4)

As can be seen in Fig. 1 the antimicrobial peptides of the invention are amphipathic molecules containing clusters of amino acids with hydrophobic (light grey) and positively charged (dark grey) side chains. These features allow them to interact with negatively charged as well as hydrophobic compounds of membrane. As consequence of the interaction the peptides incorporate into the membranes resulting in membrane destruction or pore formation.

For peptide design arginine, lysine, and histidine residues were used to create charged clusters. For generating regions of different hydrophobicity leucine, isoleucine, valine, phenylalanine, alanine, methionine, glycine, serine and threonine were used. Mean residue hydrophobicities of the hydrophobic parts of the designed peptides were calculated using the Eisenberg consensus scale of hydrophobicity (Eisenberg, 1984). Helical structure of the peptides was ensured by strong helix-forming amino acids, such as leucine, alanine and was revised using NNPREDICT for protein secondary structure prediction (Kneller et al., 1990).
D-Amino acids were incorporated into polypeptide SP7 to increase its activity against phytopathogenic fungi.

Space-filling structural models of the antimicrobial peptides were generated using Swiss-Pdb Viewer with a $\alpha$-helical structure as template (Guex and Peitsch, 1997). Amino acids with positive charged side chain are bold. Amino acids with charged or hydrophobic, hydrophilic and neutral side chain are arranged as clusters. Cluster size and orientation as well as the different amino acids composition of the clusters are important for affecting the interaction of the peptides with membranes. The N-terminus is situated at the top.

### Example 2: Determination of the minimum inhibitory concentration

In-vitro inhibition assays were performed in sterile flat-bottom 96-well plates (Greiner bio-one, Frickenhausen). Antimicrobial peptides were dissolved in 0.01% acetic acid containing 0.2% BSA (500 $\mu$g/ml), filter sterilized and stored at -20°C. For inhibition assays dilutions of peptides ranging from 5 to 200 $\mu$g/ml were prepared and 10 $\mu$l (antibacterial assay) or 20 $\mu$l (antifungal assay) of each concentration was loaded per well.

Bacteria colonies from overnight agar plate cultures were resuspended into 5ml LB-medium or HPG-medium ($OD_{600nm}$=0.08-0.1) and diluted into LB-medium/HPG-medium (1:100). 90 $\mu$l of this suspension containing about $10^5$-$10^6$ CFU/ml of tested bacteria were added to each well resulting in final peptide concentrations of 0, 0.5, 1, 2, 5, 10, and 20 $\mu$g/ml. After overnight incubation at 25 °C growth inhibition of the bacteria was analyzed by a microplate reader at $OD_{590nm}$.

Spores of fungi were collected by water washes of sporulating cultures grown on 2% malt extract agar and spore concentration was determined by microscopy. The concentration of the spores was adjusted to 1.5-2 x $10^3$ spores/ml in 2% malt extract medium and 80 $\mu$l of fungal spores were added per well. The final peptide concentrations used in the fungal inhibition assays were 0, 2, 4, 10, 20, 40, and 100 $\mu$g/ml. After overnight incubation at room temperature the plate were put on a rotary shaker (300 rpm) for further two days to ensure consistantly growth. Antifungal effects of the peptides were determined by a microplate reader (*Alternaria, Fusarium*) at $OD_{590nm}$ or by visual screening of the plates (*Botrytis, Cladosporium*).

The lowest peptide concentration leading to complete inhibition of bacterial growth or spore germination/mycelial growth (minimal inhibition concentration, MIC) was determined for each organism-peptide combination. For each peptide concentration two identical inhibition assays were performed. The results are depicted in table 1.

Table 1: Minimal inhibition concentration (MIC) for complete inhibition of bacterial growth or spore germination/mycelial growth.

| Organism | SP5 | SP6 | SP7 | SP8 | SP7-D |
|---|---|---|---|---|---|
| | | | $\mu$g/ml | | |
| Fungi | | | | | |
| *Botrytis cinerea* | > 100 | > 100 | > 100 | > 100 | 20 |
| *Alternaria alternata* | > 100 | > 100 | > 100 | > 100 | 5 |
| *Cladosporium herbarum* | 100 | 100 | 20 | 40 | 5 |
| Bacteria | | | | | |

(continued)

| Organism | SP5 | SP6 | SP7 | SP8 | SP7-D |
|---|---|---|---|---|---|
| *Clavibacter michiganensis sub. michiganensis* | 20 | > 40 | 5 | > 40 | 5 |
| *Pectobacterium carotovorum sub. carotovorum* | > 40 | > 40 | > 40 | > 40 | 40 |
| *Xanthomonas vesicatoria* | 40 | 40 | 5 | > 40 | 10 |
| *Pseudomonas syringae pv. tomato* | > 40 | 10 | 5 | > 40 | 2,5 |
| *Pseudomonas syringae pv. syringae* | 40 | 5 | 10 | > 40 | 2,5 |
| *Pseudomonas corrugata* | > 40 | 20 | 10 | > 40 | 5 |

**Example 3: Determination of hemolytic activity**

The hemolytic activity of the peptides was evaluated by determining hemoglobin release of suspensions of fresh human erythrocytes at 405 nm. Human red blood cells were centrifuged and washed three times with Tris-buffer (10 mM Tris, 150 mM NaCl, pH 7,4). Eighty $\mu$l of human red blood cells (1,5 x $10^9$/ml) were added to 20 $\mu$l of the peptides solution (peptides dissolved in 0,2% BSA, 0,01% HAc) in 96-well plates (Greiner bio-one, Frickenhausen) and incubated for 45 min at 37°C. The endconcentration of the peptides were 0, 20, 50, 100 and 200 $\mu$g/ml. After centrifugation (1500 x g, 5 min, 20°C) 30 $\mu$l aliquots of the supernatant were transferred to 96-well plates containing 100 $\mu$l water. Hemolysis was measured by absorbance at 405 nm with a microplate reader. 100% hemolysis were determined in 2% SDS. The hemolysis percentage was calculated using the following equation: % hemolysis=[(Abs$_{405\,nm}$ in the peptide solution-Abs$_{405\,nm}$ in peptides solvent)/(Abs$_{405\,nm}$ in 2% SDS-Abs$_{405\,nm}$ in peptide solvent)]$\times$100. The results are depicted in table 2.

Table 2: Hemolytic activity of the peptides, wherein the concentrations are given where at least 25% hemolysis was observed.

| SP5 | SP6 | SP7 | | SP8 | SP7-D |
|---|---|---|---|---|---|
| | | | $\mu$g/ml | | |
| >200 | >200 | >200 | | >200 | >200 |

**References**

Aerts A.M., Thevissen K., Bresseleers S.M., Sels J., Wouters P., Cammue B.P.A. and François I. E. J. A. Arabidopsis thaliana plants expressing human beta-defensin-2 are more resistant to fungal attack: functional homology between plant and human defensins. Plant Cell Rep., 2007 26(8): p. 1391-8
Alan, A.R. and Earle, E.D., Sensitivity of bacterial and fungal plant pathogens to the lytic peptides, MSI-99, magainin II, and cecropin B. Mol Plant Microbe Interact. 2002 Jul;15(7): p. 701-8
Allende, D. and T.J. McIntosh, Lipopolysaccharides in bacterial membranes act like cholesterol in eukaryotic plasma membranes in providing protection against melittin-induced bilayer lysis. Biochemistry, 2003. 42(4): p. 1101-8
Benachir, T., M. Monette, J. Grenier, and M. Lafleur, Melittin-induced leakage from phosphatidylcholine vesicles is modulated by cholesterol: A property used for membrane targeting. European Biophysics Journal with Biophysics Letters, 1997. 25(3): p. 201-210;

Cabrera M. P., Arcisio-Miranda M., Costa S.T.B., Konno K., Ruggiero J.R., Procopio J., Neto J.R. Study of the mechanism of action of anoplin, a helical antimicrobial decapeptide with ion channel-like activity, and the role of the amidated C-terminus. J. Pept Sci. 2008.14(6): p. 661-9

Casu, A., V. Pala, R. Monacelli, M. Ferro, and G. Nanni, Structure of membranes. I. Lipid composition of the erythrocyte membrane. Ital. J. Biochem., 1968. 17(2): p. 77-89

Clejan, S., T.A. Krulwich, K.R. Mondrus, and D. Seto-Young, Membrane lipid composition of obligately and facultatively alkalophilic strains of Bacillus spp. J. Bacteriol., 1986. 168(1): p. 334-40

Cruciani RA et al Antibiotic Magainins exert cytolytic activity against transformed cell lines through channel formation. Proceedings of the National Academy of Sciences (USA) 88(9): 3792-3796 (1991)

Fahrner RL, Dieckmann T, Harwig SS, Lehrer RI, Eisenberg D, Feigon J. Solution structure of protegrin-1, a broad-spectrum antimicrobial peptide from porcine leukocytes. Chem Biol. 1996;3:543-550.

Falla, T.J., D.N. Karunaratne, and R.E. Hancock, Mode of action of the antimicrobial peptide indolicidin. J. Biol. Chem., 1996. 271 (32): p. 19298-303;

Ingram, L.O., Changes in lipid composition of Escherichia coli resulting from growth with organic solvents and with food additives. Appl. Environ. Microbiol., 1977. 33(5): p. 1233-6;

Hancock, R.E. and R. Lehrer, Cationic peptides: a new source of antibiotics. Trends Biotechnol., 1998. 16(2): p. 82-8

Kokryakov VN, Harwig SS, Panyutich EA, Shevchenko AA, Aleshina GM, Shamova OV, Korneva HA, Lehrer RI. Protegrins: leukocyte antimicrobial peptides that combine features of corticostatic defensins and tachyplesins. FEBS Lett. 1993;327:231-236.

Kuzina LV et al In vitro activities of antibiotics and antimicrobial peptides against the plant pathogenic bacterium Xylella fastidiosa. Letters in Applied Microbiology 42(5): 514-520 (2006)

Lindl T, and Bauer J. (1994) Zell- und Gewebekultur. Einführung in die Grundlagen sowie ausgewählte Methoden und Anwendungen. S. 250

Matsuzaki K Magainins as paradigm for the mode of action of pore forming polypeptides. Biochimica Biophysica Acta 1376(3): 391-400 (1998).

Mosmann T. (1983) Rapid colorimetric assay for cellular growth and survival: application to proliferation and cytotoxicity assays. J. Immunol. Meth., 65, S. 55-63

Oard S.V. and Enright F.M. Expression of the antimicrobial peptides in plants to control phytopathogenic bacteria and fungi. Plant Cell Rep. 2006 25(6): p. 561-72.

Panchal RG, Smart ML, Bowser DN, Williams DA, Petrou S. Pore-forming proteins and their application in biotechnology. Curr Pharm Biotechnol. 2002;3:99-115.

Pate M. and Blazyk J. Methods for assessing the structure and function of cationic antimicrobial peptides. Methods Mol Med. 2008; 142:p. 155-73.

Piers, K.L., M.H. Brown, and R.E. Hancock, Improvement of outer membranepermeabilizing and lipopolysaccharide-binding activities of an antimicrobial cationic peptide by C-terminal modification. Antimicrob. Agents Chemother., 1994. 38(10): p. 2311-6;

Piers, K.L. and R.E. Hancock, The interaction of a recombinant cecropin/melittin hybrid peptide with the outer membrane of Pseudomonas aeruginosa. Mol. Microbiol., 1994. 12(6): p. 951-8

Schwab U., Gilligan P., Jaynes J. and Henke D. In Vitro Activities of Designed Antimicrobial Peptides against Multidrug-Resistant Cystic Fibrosis Pathogens. Antimicrob Agents Chemother., 1999. 43(6): p. 1435-1440

Sokolov Y, Mirzabekov T, Martin DW, Lehrer RI, Kagan BL. Membrane channel formation by antimicrobial protegrins. Biochim Biophys Acta. 1999;1420:23-29.

Verkleij, A.J., R.F. Zwaal, B. Roelofsen, P. Comfurius, D. Kastelijn, and L.L. van Deenen, The asymmetric distribution of phospholipids in the human red cell membrane. A combined study using phospholipases and freeze-etch electron microscopy. Biochim. Biophys. Acta, 1973. 323(2): p. 178-93;

Zasloff M Magainins, a class of antimicrobial peptides from Xenopus skin: isolation, characterization of two active forms, and partial cDNA sequence of a precursor. Proceedings of the National Academy of Sciences (USA) 84(15): 5449-5453 (1987)

Zasloff, M., Antimicrobial peptides of multicellular organisms. Nature, 2002, 415(6870): p. 389-95

**Claims**

1. A peptide comprising or consisting of a sequence of formula I

# A-B-C-D-E-F (formula I), wherein A is a peptide consisting of three hydrophobic amino acid residues;
   B is an amino acid residue selected from the group consisting of hydrophobic and basic amino acid residues;
   C is a peptide consisting of two hydrophobic amino acid residues;
   D is a peptide consisting of two basic amino acid residues;
   E is a peptide consisting of two hydrophobic amino acid residues; and
   F is a peptide consisting of two basic amino acid residues;

   or a peptidomimetic thereof
   wherein the basic amino acid residues are selected from the group consisting of arginine, lysine and histidine;
   wherein the hydrophobic amino acid residues are selected from the group consisting of leucine, alanine, valine, isoleucine, methionine and phenylalanine;
   and wherein said peptide or peptidomimetic has antimicrobial activity.

2. The peptide or peptidomimetic of claim 1, comprising or consisting of the following amino acid sequence:

# A1-A2-A3-A4-A5-A6-A7-A8-A9-A10-A11-A12 (formula II), wherein A1, A2, A3, A5, A6, A9, A10 A4 are hydrophobic amino acid residues; is an amino acid residue selected from the group consisting of hydrophobic and basic amino acid residues;
   A7, A8, A11,A12 are basic amino acid residues,

3. The peptide or peptidomimetic according to any one of claims 1 or 2, wherein the peptide comprises or consists of a sequence selected from the group consisting of LLIAAFKKLVKK, ALAHFLKKAIKK, LLIKFLKRFIKH and LLIRAAKKFIKK.

4. The peptide or peptidomimetic according to any one of claims 1 to 3, wherein the peptide comprises one or several D-amino acids.

5. The peptide or peptidomimetic according to claim 4, wherein the peptide comprises or consists of the sequence LL*I*KF*LKR*F/KH, wherein the amino acid residues represented in bold and italics represent D-amino acid residues.

6. The peptide or peptidomimetic according to any one of claims 1 to 5, wherein the antimicrobial activity is directed against at least one organism selected from the group of plant pathogens consisting of *Pseudomonas syringae pv.*

*syringae, Pseudomonas syringae pv. tomato, Pseudomonas corrugata, Pectobacterium carotovorum sub. carotovorum, Clavibacter michiganensis sub. michiganensis, Xanthomonas vesicatoria, Erwinia amylovora, Botrytis cinerea, Alternaria alternata* and *Cladosporium herbarum* and from the group of human pathogens consisting of *Enterobacter cloacae, Yersinia enterocolitica, Klebsiella pneumoniae, Klebsiella oxytoca, Pseudomonas aeruginosa, Staphylococcus aureus, Staphylococcus epidermidis* and multi resistant strains.

7.  A nucleic acid molecule encoding a peptide according to any one of claims 1 to 6.

8.  A vector comprising the nucleic acid molecule of claim 7.

9.  A host cell comprising the nucleic acid molecule of claim 7 or the vector of claim 8.

10. A method for producing a peptide or peptidomimetic according to any one of claims 1 to 6, comprising culturing the host of claim 9 under suitable conditions and isolating the peptide produced.

11. A composition comprising a peptide or peptidomimetic according to any one of claims 1 to 6, the nucleic acid molecule of claim 7, the vector of claim 8 or the host cell of claim 9.

12. The composition of claim 11, wherein the composition is selected from the group consisting of a pharmaceutical composition or a plant protective composition; and optionally further comprises a suitable carrier and/or diluent.

13. The peptide or peptidomimetic according to any one of claims 1 to 6, the nucleic acid molecule of claim 70, the vector of claim 8 or the host cell of claim 9 for use in treating infectious diseases.

|       |                   |       | HYDROPHOBICITIES<br>OF HYDROPHOBIC AREAS | PROTEIN SECONDARY<br>STRUCTURE PREDICTION |
|-------|-------------------|-------|---------------|-----------------------|
| SP5:  | LLIAAFKKLVKK-NH2  | (+4)  | 3,97          | -HHHHHHHHH--          |
| SP6:  | ALAHFLKKAIKK-NH2  | (+5)  | 3,15          | Prediction not possible |
| SP7:  | LLIKFLKRFIKH-NH2  | (+5)  | 4,27          | -HHHHHHHHH--          |
| SP8:  | LLIRAAKKFIKK-NH2  | (+5)  | 3,63          | HHHHHHHHHH--          |

**Figure 1**

**Figure 2**

**Europäisches Patentamt**
**European Patent Office**
**Office européen des brevets**

## EUROPEAN SEARCH REPORT

Application Number

EP 08 01 6808

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 7 381 704 B2 (OWEN DONALD R [US]) 3 June 2008 (2008-06-03) * sequences 8, 9, 152 * | 1-4,6-13 | INV. A61K38/04 C07K7/08 |
| X | WO 96/03519 A1 (DEMETER BIOTECH LTD [US]; US AGRICULTURE [US]) 8 February 1996 (1996-02-08) * sequences 69, 70, 71 * * page 27 * | 1-4,6-13 | |
| X | US 2008/153748 A1 (JAYNES JESSE [US]) 26 June 2008 (2008-06-26) * sequences 20, 23-25 * * table 1 * * paragraph [0030] * | 1-4,6-13 | |
| X | WO 99/54474 A2 (GLAXO GROUP LTD [GB]; ARIGONI FABRIZIO [CH]; EDGERTON MICHAEL DAVID [U) 28 October 1999 (1999-10-28) * figure 2a * | 1-4,6-13 | |
| X | KICHLER ANTOINE ET AL: "Characterization of the gene transfer process mediated by histidine-rich peptides." JOURNAL OF MOLECULAR MEDICINE (BERLIN, GERMANY) FEB 2007, vol. 85, no. 2, February 2007 (2007-02), pages 191-201, XP019467088 ISSN: 0946-2716 * figure 3 * * tables 2,3 * | 1-4,6-13 | TECHNICAL FIELDS SEARCHED (IPC) C07K |

-/--

~~The present search report has been drawn up for all claims~~

| Place of search | Date of completion of the search | Examiner |
|---|---|---|
| The Hague | 27 November 2008 | Schmitz, Till |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 08 01 6808

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | LEE KEUN-HYEUNG: "Development of short antimicrobial peptides derived from host defense peptides or by combinatorial libraries" CURRENT PHARMACEUTICAL DESIGN, BENTHAM SCIENCE PUBLISHERS, SCHIPHOL, NL, vol. 8, no. 9, 1 January 2002 (2002-01-01), pages 795-813, XP002422399 ISSN: 1381-6128 * figure 1; table 1 * ----- | | |
| A | KIYOTA T ET AL: "The effect of D-amino acid-containing basic peptides with different hydrophobicity on the antimicrobial and cytotoxic activity" BULLETIN OF THE CHEMICAL SOCIETY OF JAPAN, CHEMICAL SOCIETY OF JAPAN, TOKYO, JP, vol. 73, no. 10, 1 January 2000 (2000-01-01), pages 2363-2370, XP002422400 ISSN: 0009-2673 * the whole document * ----- | | TECHNICAL FIELDS SEARCHED (IPC) |
| A,D | HANCOCK R E W ET AL: "Cationic peptides: a new source of antibiotics" TRENDS IN BIOTECHNOLOGY, ELSEVIER PUBLICATIONS, CAMBRIDGE, GB, vol. 16, no. 2, 1 February 1998 (1998-02-01), pages 82-88, XP004107047 ISSN: 0167-7799 * the whole document * ----- | | |

-/--

~~The present search report has been drawn up for all claims~~

| Place of search | Date of completion of the search | Examiner |
|---|---|---|
| The Hague | 27 November 2008 | Schmitz, Till |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 08 01 6808

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | ZASLOFF M: "ANTIMICROBIAL PEPTIDES OF MULTICELLULAR ORGANISMS" NATURE, NATURE PUBLISHING GROUP, LONDON, UK, vol. 415, no. 6870, 24 January 2002 (2002-01-24), pages 389-395, XP001027353 ISSN: 0028-0836 * the whole document * | | |

TECHNICAL FIELDS
SEARCHED      (IPC)

~~The present search report has been drawn up for all claims~~

| Place of search | Date of completion of the search | Examiner |
|---|---|---|
| The Hague | 27 November 2008 | Schmitz, Till |

CATEGORY OF CITED DOCUMENTS

X : particularly relevant if taken alone
Y : particularly relevant if combined with another
     document of the same category
A : technological background
O : non-written disclosure
P : intermediate document

T : theory or principle underlying the invention
E : earlier patent document, but published on, or
     after the filing date
D : document cited in the application
L : document cited for other reasons

& : member of the same patent family, corresponding
     document

EPO FORM 1503 03.82 (P04C01)

Europäisches
Patentamt
European
Patent Office
Office européen
des brevets

**Application Number**

EP 08 01 6808

---

**CLAIMS INCURRING FEES**

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

---

**LACK OF UNITY OF INVENTION**

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

see annex

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

The Search Division considers that the present European patentapplication does not comply with the requirements of unity of invention and relates to severalinventions or groups of inventions, namely:

    1. claims: 1-4, 6-14 (partially)

        An antimicrobial peptide as defined in SEQ ID NO:1 (LLIAAFKKLVKK). Furthermore nucleic acids, vectors, host cells, methods, compositions relating thereto.
            ---

    2. claims: 1-4, 6-14 (partially)

        as invention 1, but relating to SEQ ID NO:2 (ALAHFLKKAIKK).
            ---

    3. claims: 1-4, 6-14 (partially); 5 (completely)

        As invention 1, but relating to SEQ ID NO:3 (LLIKFLKRFIKH).
            ---

    4. claims: 1-4, 6-14 (partially)

        As invention 1, but relating to SEQ ID NO:4 (LLIRAAKKFIKK).
            ---

## ANNEX TO THE EUROPEAN SEARCH REPORT
## ON EUROPEAN PATENT APPLICATION NO.

EP 08 01 6808

This annex lists the patent family members relating to the patent documents cited in the above-mentioned European search report.
The members are as contained in the European Patent Office EDP file on
The European Patent Office is in no way liable for these particulars which are merely given for the purpose of information.

27-11-2008

| Patent document cited in search report | | Publication date | Patent family member(s) | | Publication date |
|---|---|---|---|---|---|
| US 7381704 | B2 | 03-06-2008 | AU<br>CA<br>EP<br>JP<br>WO<br>US | 2002255952 A1<br>2441562 A1<br>1379265 A2<br>2005506300 T<br>02079408 A2<br>2003109452 A1 | 15-10-2002<br>10-10-2002<br>14-01-2004<br>03-03-2005<br>10-10-2002<br>12-06-2003 |
| WO 9603519 | A1 | 08-02-1996 | AU<br>AU<br>CA<br>EP<br>JP<br>WO | 3199595 A<br>3199695 A<br>2195625 A1<br>0781347 A1<br>10507069 T<br>9603522 A1 | 22-02-1996<br>22-02-1996<br>08-02-1996<br>02-07-1997<br>14-07-1998<br>08-02-1996 |
| US 2008153748 | A1 | 26-06-2008 | NONE | | |
| WO 9954474 | A2 | 28-10-1999 | AU | 3709099 A | 08-11-1999 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **Black, S.D. ; Mould, D.R.** Development of Hydrophobicity Parameters to Analyze Proteins Which Bear Post- or Cotranslational Modifications. *Anal. Biochem.,* 1991, vol. 193, 72-82
- **Aerts A.M. ; Thevissen K. ; Bresseleers S.M. ; Sels J. ; Wouters P. ; Cammue B.P.A. ; François I. E. J. A.** Arabidopsis thaliana plants expressing human beta-defensin-2 are more resistant to fungal attack: functional homology between plant and human defensins. *Plant Cell Rep.,* 2007, vol. 26 (8), 1391-8
- **Alan, A.R. ; Earle, E.D.** Sensitivity of bacterial and fungal plant pathogens to the lytic peptides, MSI-99, magainin II, and cecropin B. *Mol Plant Microbe Interact.,* July 2002, vol. 15 (7), 701-8
- **Allende, D. ; T.J. McIntosh.** Lipopolysaccharides in bacterial membranes act like cholesterol in eukaryotic plasma membranes in providing protection against melittin-induced bilayer lysis. *Biochemistry,* 2003, vol. 42 (4), 1101-8
- **Benachir, T. ; M. Monette ; J. Grenier ; M. Lafleur.** Melittin-induced leakage from phosphatidylcholine vesicles is modulated by cholesterol: A property used for membrane targeting. *European Biophysics Journal with Biophysics Letters,* 1997, vol. 25 (3), 201-210
- **Cabrera M. P. ; Arcisio-Miranda M. ; Costa S.T.B. ; Konno K. ; Ruggiero J.R. ; Procopio J. ; Neto J.R.** Study of the mechanism of action of anoplin, a helical antimicrobial decapeptide with ion channel-like activity, and the role of the amidated C-terminus. *J. Pept Sci.,* 2008, vol. 14 (6), 661-9
- **Casu, A. ; V. Pala ; R. Monacelli ; M. Ferro ; G. Nanni.** Structure of membranes. I. Lipid composition of the erythrocyte membrane. *Ital. J. Biochem.,* 1968, vol. 17 (2), 77-89
- **Clejan, S. ; T.A. Krulwich ; K.R. Mondrus ; D. Seto-Young.** Membrane lipid composition of obligately and facultatively alkalophilic strains of Bacillus. *J. Bacteriol.,* 1986, vol. 168 (1), 334-40
- **Cruciani RA et al.** Antibiotic Magainins exert cytolytic activity against transformed cell lines through channel formation. *Proceedings of the National Academy of Sciences (USA),* 1991, vol. 88 (9), 3792-3796

- **Fahrner RL ; Dieckmann T ; Harwig SS ; Lehrer RI ; Eisenberg D ; Feigon J.** Solution structure of protegrin-1, a broad-spectrum antimicrobial peptide from porcine leukocytes. *Chem Biol.,* 1996, vol. 3, 543-550
- **Falla, T.J. ; D.N. Karunaratne ; R.E. Hancock.** Mode of action of the antimicrobial peptide indolicidin. *J. Biol. Chem.,* 1996, vol. 271 (32), 19298-303
- **Ingram, L.O.** Changes in lipid composition of Escherichia coli resulting from growth with organic solvents and with food additives. *Appl. Environ. Microbiol.,* 1977, vol. 33 (5), 1233-6
- **Hancock, R.E. ; R. Lehrer.** Cationic peptides: a new source of antibiotics. *Trends Biotechnol.,* 1998, vol. 16 (2), 82-8
- **Kokryakov VN ; Harwig SS ; Panyutich EA ; Shevchenko AA ; Aleshina GM ; Shamova OV ; Korneva HA ; Lehrer RI.** Protegrins: leukocyte antimicrobial peptides that combine features of corticostatic defensins and tachyplesins. *FEBS Lett.,* 1993, vol. 327, 231-236
- **Kuzina LV et al.** In vitro activities of antibiotics and antimicrobial peptides against the plant pathogenic bacterium Xylella fastidiosa. *Letters in Applied Microbiology,* 2006, vol. 42 (5), 514-520
- **Lindl T ; Bauer J.** Zell- und Gewebekultur. *Einführung in die Grundlagen sowie ausgewählte Methoden und Anwendungen,* 1998, 250
- **Matsuzaki K.** Magainins as paradigm for the mode of action of pore forming polypeptides. *Biochimica Biophysica Acta,* vol. 1376 (3), 391-400
- **Mosmann T.** Rapid colorimetric assay for cellular growth and survival: application to proliferation and cytotoxicity assays. *J. Immunol. Meth.,* 1983, vol. 65, 55-63
- **Oard S.V. ; Enright F.M.** Expression of the antimicrobial peptides in plants to control phytopathogenic bacteria and fungi. *Plant Cell Rep.,* 2006, vol. 25 (6), 561-72
- **Panchal RG ; Smart ML ; Bowser DN ; Williams DA ; Petrou S.** Pore-forming proteins and their application in biotechnology. *Curr Pharm Biotechnol.,* 2002, vol. 3, 99-115
- **Pate M. ; Blazyk J.** Methods for assessing the structure and function of cationic antimicrobial peptides. *Methods Mol Med.,* 2008, vol. 142, 155-73

- **Piers, K.L. ; M.H. Brown ; R.E. Hancock.** Improvement of outer membranepermeabilizing and lipopolysaccharide-binding activities of an antimicrobial cationic peptide by C-terminal modification. *Antimicrob. Agents Chemother.,* 1994, vol. 38 (10), 2311-6
- **Piers, K.L. ; R.E. Hancock.** The interaction of a recombinant cecropin/melittin hybrid peptide with the outer membrane of Pseudomonas aeruginosa. *Mol. Microbiol.,* 1994, vol. 12 (6), 951-8
- **Schwab U. ; Gilligan P. ; Jaynes J. ; Henke D.** In Vitro Activities of Designed Antimicrobial Peptides against Multidrug-Resistant Cystic Fibrosis Pathogens. *Antimicrob Agents Chemother,* 1999, vol. 43 (6), 1435-1440
- **Sokolov Y ; Mirzabekov T ; Martin DW ; Lehrer RI ; Kagan BL.** Membrane channel formation by antimicrobial protegrins. *Biochim Biophys Acta,* 1999, vol. 1420, 23-29
- **Verkleij, A.J. ; R.F. Zwaal ; B. Roelofsen ; P. Comfurius ; D. Kastelijn ; L.L. van Deenen.** The asymmetric distribution of phospholipids in the human red cell membrane. A combined study using phospholipases and freeze-etch electron microscopy. *Biochim. Biophys. Acta,* 1973, vol. 323 (2), 178-93
- **Zasloff M.** Magainins, a class of antimicrobial peptides from Xenopus skin: isolation, characterization of two active forms, and partial cDNA sequence of a precursor. *Proceedings of the National Academy of Sciences (USA),* 1987, vol. 84 (15), 5449-5453
- **Zasloff, M.** Antimicrobial peptides of multicellular organisms. *Nature,* 2002, vol. 415 (6870), 389-95